# EUROPEAN PATENT SPECIFICATION

(11) **EP 2 566 852 B1**
(45) Date of publication and mention of the grant of the patent: **24.12.2014**
(21) Application number: 11775537.1
(22) Date of filing: 27.04.2011
(51) Int. Cl.: A62D 3/38, B01J 31/18, C02F 1/72, C11D 3/39

(54) **CARBON SUPPORTED TETRAAMIDO MACROCYCLIC LIGAND CATALYTIC ACTIVATORS AND METHODS FOR MAKING THE SAME**
KOHLENSTOFFGESTÜTZTE MAKROZYKLISCHE UND KATALYTISCHE TETRAAMIDO-LIGANDENAKTIVATOREN SOWIE HERSTELLUNGSVERFAHREN DAFÜR
ACTIVATEURS CATALYTIQUES À LIGANDS MACROCYCLIQUES TÉTRAAMIDO, SUPPORTÉS SUR CARBONE, ET LEURS PROCÉDÉS DE FABRICATION

(30) Priority: 27.04.2010 US 343330 P
(43) Date of publication of application: 13.03.2013
(73) Proprietor: Carnegie Mellon University, Pittsburgh, PA 15213 (US)
(72) Inventor: COLLINS, Terrence, J., Pittsburgh, PA 15206-1716 (US); HORWITZ, Colin, P., Pittsburgh, PA 15206 (US); WASHBURN, Newell, R., Pittsburgh, PA 15217 (US); ELLIS, William, Ithaca, NY 14850 (US); ROY, Riddhi, Pittsburgh, PA 15217 (US)
(74) Representative: Barton, Matthew Thomas
(86) International application number: PCT/US2011/034193
(87) International publication number: WO 2011/137190

(56) References cited:
- US-A- 6 054 580
- US-A1- 2010 010 285
- US-B1- 6 241 779

## Description

### CROSS-REFERENCE TO RELATED APPLICATIONS

This application claims the benefit of U. S. Provisional Application Serial No. 61/343,330 filed April 27, 2010.

### BACKGROUND OF THE INVENTION

### Field of the Invention

The various embodiments of the invention relate to tetraamido macrocyclic ligand catalytic activators bound to a carbon support and methods of binding the ligand catalysts on the supports.

### Background of the Related Art

Iron-based enzymes catalyze oxidation reactions using oxygen and hydrogen peroxide, but few synthetic analogues can match their efficacy and stability. The series of small molecule non-heme iron complexes, tetraamido macrocyclic ligand catalytic activators are proving to be highly effective mimics of the peroxidase enzymes. (Peroxidases, H. B. Dunford, Adv. Inorg. Biochem., 1982, vol. 4, pp. 41-80) (H. B. Dunford, Heme Peroxidases, Wiley-VCH, New York, Chichester, Weinheim, 1999.)

Tetraamido macrocyclic ligand catalytic activators of hydrogen peroxide are also showing reactivity similar to cytochrome-P450 enzymes. These latter enzymes usually make coordinated peroxide from oxygen at their active site and then proceed to use it, but can be short-circuited (relieve the requirement to reduce oxygen) by the use of various oxidants, particularly but not exclusively oxidants generated by partial reduction of oxygen such as hydrogen peroxide (H₂O₂) (Heme-containing oxygenases, M. Sono, M. P. Roach, E. D. Coulter, J. H. Dawson, Chem. Rev., 1996, vol. 96, pp. 2841-2887.) (Geometric and electronic structure/function correlations in non-heme iron enzymes, E. I. Solomon, T. C. Brunold, M. I. Davis, J. N. Kemsley, S.-K. Lee, N. Lehnert, F. Neese, A. J. Skulan, Y.-S. Yang, J. Zhou, Chem. Rev., 2000, vol. 100, pp. 235-349.) (Mechanism of oxidation reactions catalyzed by cytochrome P450 enzymes, B. Meunier, S. P. de Visser, S. Shaik, Chem. Rev., 2004, vol. 104, pp. 3947-3980.). In the precatalyst forms of tetraamido macrocyclic ligand catalysts, metal ions, usually iron(III) ions are bonded to the four deprotonated amide-N atoms of macrocyclic tetraamide ligands. Tetraamido macrocyclic ligand catalytic activators catalyze peroxide-based and other oxidant-based oxidation processes to oxidize a range of substrates, exhibiting high reactivity that is similar to the peroxidase enzymes themselves. While the relatively environmentally compatible and inexpensive hydrogen peroxide usually serves as the ultimate source of the oxygen atom, as happens with peroxidase enzymes, tetraamido macrocyclic ligand catalytic activators remain fairly stable under catalytic conditions without the protection of the protein environment afforded to the active sites in the enzymes such that they are able to achieve high turnover numbers until they eventually degrade under the potent oxidizing conditions. (TAML oxidant activators: A new approach to the activation of hydrogen peroxide for environmentally significant problems, T. J. Collins, Acc. Chem. Res., 2002, vol. 35, pp. 782-790.) (Little green molecules, T. J. Collins, C. Walter, Scientific American, 2006, vol. 294, pp. 83-88, 90.)

The suitability of tetraamido macrocyclic ligand catalytic activator/peroxide processes for removing certain contaminants from water has been tested now for a wide range of organic pollutants, often ones that are unreactive, and the technical performance is proving to be impressive (TAML oxidant activators: A new approach to the activation of hydrogen peroxide for environmentally significant problems, T. J. Collins, Acc. Chem. Res., 2002, vol. 35, pp. 782-790.). These activators catalyze the chemistry of a variety of peroxides. The reactions typically take place at room temperature under ambient conditions, although there is a pH dependence of the reactivity with highest rates being found under basic conditions near pH 10 for many members of the catalyst family.

In general, removal of contaminants from solution or from vapors, as in removal of volatile organic compounds from water vapors (Removal of VOCs from humidified gas streams using activated carbon cloth, Mark P. Cala, Mark J. Rood and Susan M. Larson, Gas Separation & Purification, Vol: 10 Issue: 2 ISSN: 0950-4214 Date: June 1996, pp 117 - 121), is often achieved by passing the fluid phase through porous or high surface area carbon solids. One commonly used class of carbon is activated carbon (Activated Carbon by H. Marsh and F. R. Reinoso; 2005; Elsevier Science; ISBN-10: 0080444636), which has subclasses that include granular organic carbon and powdered activated carbon. Powdered activated carbon has smaller particles and larger surface area, and it generally provides improved adsorption compared to granular organic carbon. Removal of contaminants is achieved through selective adsorption of the contaminants. Porous carbon solids have been used to remove organometallic and metallorganic species from solution, and many such species are known to have strong affinities for carbon surfaces. (Chemical process for removing organometallic compounds from water, U.S. Patent No. 5,332,509) In such removal applications, commonly used carbons include activated carbon and charcoal, but highly crystalline graphite powders as well as amorphous carbons, such as carbon black, could also be used.

Hybrid strategies for removing contaminants from solution have been developed that involve oxidation or reduction reactions in addition to contaminant adsorption or immobilization. For example, electrocoagulation has been used to precipitate organic dyes from solution which is performed through anode oxidation coupled with hydroxide production leading to sludge formation. (Anionic reactive dye removal from aqueous solution using a new adsorbent-Sludge generated in removal of heavy metal by electrocoagulation, Golder et al., Chemical Engineering Journal, Volume 122, Issues 1-2, 1 September 2006, pp 107-115) An alternate approach is to use activated carbon as an electrode in producing hydrogen peroxide *in situ* to degrade oxidatively contaminants (Wastewater Treatment Using Electrolysis With Activated Carbon Cathode, Kawahata M, Price K.S., U.S. Patent No. 3,793,173, (1974)). A two-step desulfurization process for hydrocarbon fuels has been reported in which tetraamido macrocyclic ligand catalysts are used in the presence of oxygen, not peroxide, to oxidize thiophenic compounds to corresponding sulfone or sulfoxide species that adsorb readily to activated carbon. (A novel method for oxidative desulfurization of liquid hydrocarbon fuels based on catalytic oxidation using molecular oxygen coupled with selective adsorption. Ma X, Zhou A, Song C, Catalysis Today, Volume 123, Issues 1-4, 30 May 2007, pp. 276-284)

Adsorption of organometallic and metal-organic catalysts to carbon surfaces or association with carbon supports has been reported as a means to immobilize them but does have limitations in terms of versatility and applicability across classes of catalysts since there is no guarantee that they will retain activity when adsorbed to a surface. There are few examples of catalysts that remain catalytically active following attachment or binding to a support. Examples of catalysts that have been shown to be active include transition metal complexes with phthalocyanines and porphyrins bound on activated carbon surfaces that were used to reduce gas-phase CO₂ (Electrochemical Reduction of CO2 with Transition Metal Phthalocyanine and Porphyrin Complexes Supported on Activated Carbon Fibers, T. V. Magdesieva et al., J. Electrochem. Soc., Volume 149, Issue 6, pp. D89-D95 (June 2002)), a mixed aqueous- and solid-phase catalyst system that immobilizes organic Rh complexes capable of hydroformylation reactions, (U.S. Patent No. 4,994,427, Supported aqueous phase organometallic catalyst useful for hydroformylation and other reactions, and a method for its preparation), and numerous examples of organometallic catalysts adsorbed to solid supports and super critical CO₂ that have been used to effect chemical transformations, such as styrene hydrovinylation. (Recent advances in catalyst immobilization using supercritical carbon dioxide, Leitner W., Pure Appl. Chem., Vol. 76, No. 3, pp. 635-644, (2004))

Strategies for incorporating tetraamido macrocyclic ligand catalysts in solid supports have been reported by others. Wang et al. developed a method in which tetraamido macrocyclic ligand catalysts were incorporated into a pyrolytic graphite electrode impregnated with sodium alginate and demonstrated electrochemical activity of the tetraamido macrocyclic ligand catalysts in use as a sensor for H₂O₂. (Wang J; Sun, H.; Zhao, X.S, Electrochemical catalysis and stability of tetraamido macrocyclic ligands iron immobilized on modified pyrolytic graphite electrode, Catalysis Today, 158, pp. 263-268, (2010)) A method for preparing electrocatalysts in the production of hydrogen peroxide from water has also been reported (Electroactivated film with polymer gel electrolyte, U.S. Patent No. 7,842,637; Electroactivated film with layered structure, U.S. published Application US 2009/0288946; Electroactivated film with immobilized peroxide activating catalyst U.S. published Application US 2009/0291844; Electroactivated film with electrocatalyst-enhanced carbon electrode, U.S. published Application US 2009/0288945). In the embodiments in these applications that involve the tetraamido macrocyclic ligand catalysts, the catalyst is held in close proximity to carbon or other electrodes by a polyelectrolyte layer and activates peroxide formed at the electrodes to form potent oxidizing species, layer that are not present in the inventions described here.

### SUMMARY OF THE INVENTION

According to the present invention, there is provided an active oxidant system comprising:
a macrocyclic tetradentate ligand having the structure 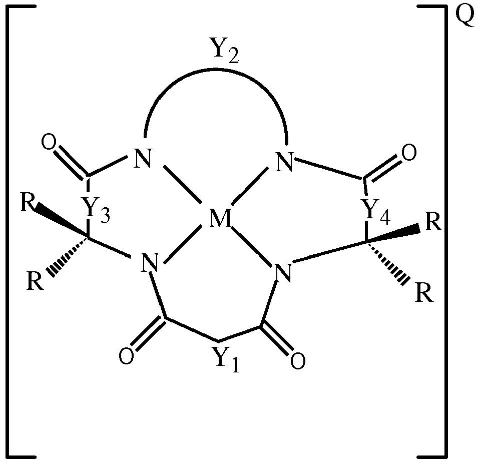 wherein Y₁, Y₃ and Y₄ each represents a bridging group, having zero, one, two or three carbon containing nodes for substitution, and Y₂ is a bridging group having at least one carbon containing node for substitution, each said node containing a C(R) or a C(R)₂ unit and each R substituent is the same or different from the remaining R substituents and (i) is selected from the group consisting of alkyl, cycloalkyl, cycloalkenyl, alkenyl, aryl, alkynyl, alkylaryl, halogen, alkoxy, or phenoxy, CH₂CF₃, CF₃ and combinations thereof, or (ii) form a substituted or unsubstituted benzene ring of which two carbon atoms in the ring form nodes in the Y unit, or (iii) together with a paired R substituent bound to the same carbon atom form a cycloalkyl or a cycloalkenyl ring, which may include an atom other than carbon, e.g., cyclopropyl, cyclobutyl, cyclopentyl, or a cyclohexyl ring; M is a transition metal with oxidation states of I, II, III, IV, V, VI, VII or VIII or selected from Groups 3, 4, 5, 6, 7, 8, 9, 10 and 11 of the Periodic Table of the Elements; and, Q is any counterion which would balance the charge of the compound on a stoichiometric basis; characterized in that the macrocyclic tetradentate ligand is bound to a carbon-containing support selected from the group consisting of activated carbon, amorphous carbon, and charcoal; and the system further comprises a source of an oxidant.

Advantageously, the oxidant is selected from the group consisting of O-atom transfer oxidants, molecular oxygen, sources of oxygen, ozone, hydrogen peroxide, hydrogen peroxide adducts, t-butyl hydroperoxide, cumyl hydroperoxide, compounds capable of producing hydrogen peroxide in aqueous solution, organic peroxides, perborates, percarbonates, persulfates, perphosphates, persilicates, hypochlorite, peracids, and combinations thereof.

Preferably, the counterion Q is selected from the group consisting of tetraarylphosphonium, bis- (triphenylphosphorananylidene)-ammonium, and tetraalkylammonium cations.

According to the present invention there is also provided a method for removing oxidizable contaminants from an aqueous medium comprising: exposing an aqueous medium believed to contain oxidizable contaminants to a tetraamido macrocyclic ligand metal catalytic activator bound to a carbon-containing support for a period of time sufficient to oxidize the contaminants, wherein the tetraamido macrocyclic metal ligand comprises a salt of 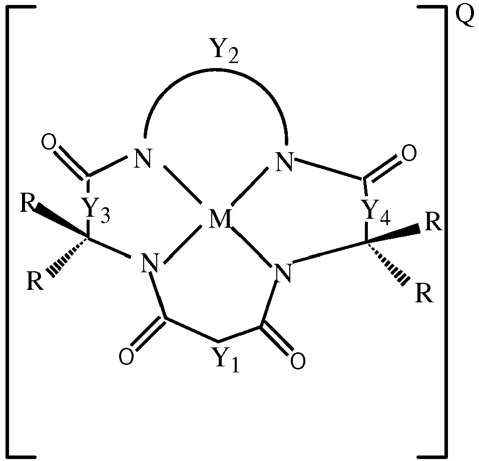 wherein Y₁, Y₃ and Y₄ each represents a bridging group, having zero, one, two or three carbon containing nodes for substitution, and Y₂ is a bridging group having at least one carbon containing node for substitution, each said node containing a C(R) or a C(R)₂ unit and each R substituent is the same or different from the remaining R substituents and ( i ) is selected from the group consisting of alkyl, cycloalkyl, cycloalkenyl, alkenyl, aryl, alkynyl, alkylaryl, halogen, alkoxy, or phenoxy, CH₂CF₃, CF₃ and combinations thereof, or (ii) form a substituted or unsubstituted benzene ring of which two carbon atoms in the ring form nodes in the Y unit, or (iii) together with a paired R substituent bound to the same carbon atom form a cycloalkyl or a cycloalkenyl ring, which may include an atom other than carbon, e.g., cyclopropyl, cyclobutyl, cyclopentyl, or a cyclohexyl ring; M is a transition metal with oxidation states of I, II, III, IV, V, VI, VII or VIII or selected from Groups 3, 4, 5, 6, 7, 8, 9, 10 and 11 of the Periodic Table of the Elements; and, Q is any counterion which would balance the charge of the compound on a stoichiometric basis.

Conveniently, the carbon-containing support is selected from the group consisting of activated carbon, amorphous carbon, and carbon black, charcoal.

Preferably, the oxidizable contaminants comprise: aromatic groups, conjugated pi systems, natural and synthetic hormones, pesticides, and dyes.

Advantageously, the oxidizable contaminants comprise pathogens.

Preferably, the method further comprises (a) providing a plurality of discrete filtration surfaces spaced along a feed stream, each said filtration surface comprising an tetraamido macrocyclic metal ligand catalytic activator bound to the carbon-containing support; (b) adding an oxidant to the feed stream; and, (c) passing the oxidant containing feed stream through at least one of the filtration surfaces.

Conveniently, the oxidant is selected from the group consisting of O-atom transfer oxidants, molecular oxygen, sources of oxygen, ozone, hydrogen peroxide, hydrogen peroxide adducts, t-butyl hydroperoxide, cumyl hydroperoxide, compounds capable of producing hydrogen peroxide in aqueous solution, organic peroxides, perborates, percarbonates, persulfates, perphosphates, persilicates, hypochlorite, peracids, and combinations thereof.

Advantageously, the feed stream flows past the carbon supported catalytic activator.

### BRIEF DESCRIPTION OF FIGURES

Various features and characteristics of the non-limiting and non-exhaustive embodiments disclosed and described in this specification may be better understood by reference to the accompanying figures, in which:

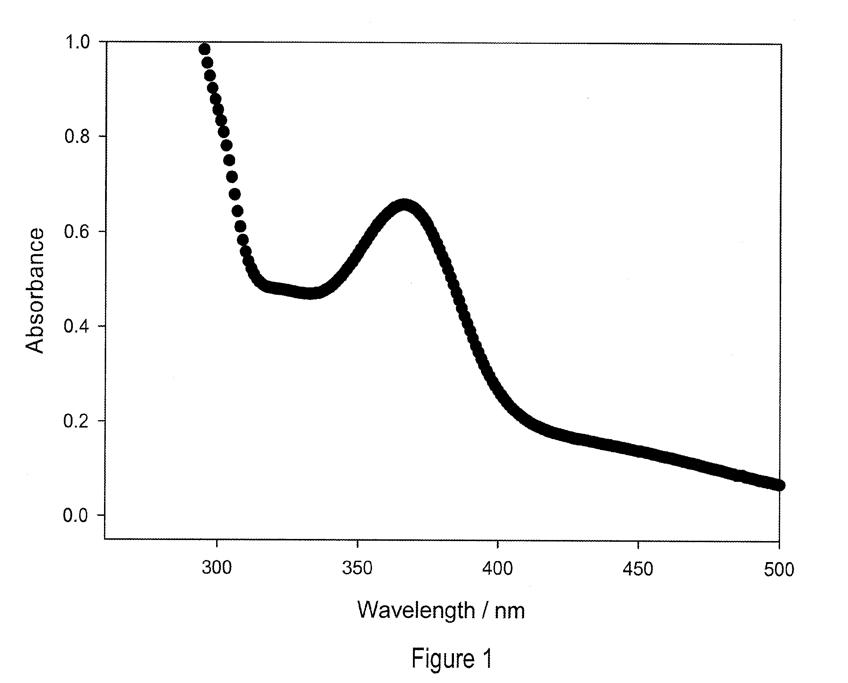

Figure 1 shows a sample UV/Vis absorption spectrum of NaFeB* in methanol with absorption peak at 365 nm.
Figure 2 is a scanning electron microscope image of the granular activated carbon used in these studies.

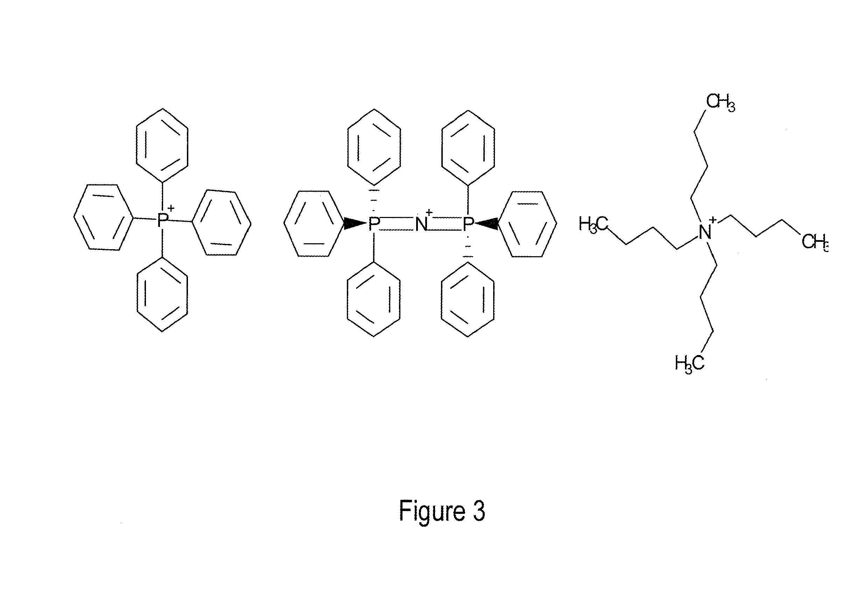

Figure 3 shows the chemical structures of three countercations of the FeB* anion (PPh₄⁺, PNP⁺ and Bu₄N⁺) used in preparation of various embodiments of the catalyst-loaded carbon supports.
Figure 4 is a collection of bleaching curves plotted as concentration of Orange (II) as a function of time for experiments running over 5 days using PPh₄FeB* on 12x30 OLC activated carbon (Calgon Corporation, Inc.). Reaction conditions: [Or(II)] = 4 × 10⁻⁵M, [H₂O₂] = 2.3 × 10⁻³ M, 50 mg activated carbon containing 2.1x10⁻⁶ moles PPh₄FeB*, 0.01 M pH 7.0 phosphate buffer, Temperature= 25°C. All curves display exponential decays of the Or(II) absorbance signal. Variability between runs is more common with the smaller sample size used in these particular measurements.
Figure 5 is a graph showing the catalytic activity of NaFeB* (solid curve) and PPh4FeB* (dashed curve) bound to OLC 12x30 activated carbon (Calgon Carbon, Inc.) in separate experiments on the same samples performed over a period of 40 days. Catalytic activity was assessed by measuring the initial rate of decay of absorption at 485 nm light scaled per mole of catalyst on the carbon support.
Figure 6 is a graph showing the results of a test of demetallation of catalytic activator bound to a 12x30 OLC that was exposed to 0.1 M phosphate buffer solution over 5 days. The concentration of [Or(II)] bleached in 450 seconds is shown for normal homogeneous NaFeB* ( * ) compared with supported NaFeB* ( 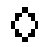 ) for days 1 - 5.

The reader will appreciate the foregoing details, as well as others, upon considering the following detailed description of various non-limiting and non-exhaustive embodiments according to the present disclosure.

### DETAILED DESCRIPTION OF THE INVENTION

Also, any numerical range recited in this specification is intended to include all sub-ranges of the same numerical precision subsumed within the recited range. For example, a range of "1.0 to 10.0" is intended to include all sub-ranges between (and including) the recited minimum value of 1.0 and the recited maximum value of 10.0, that is, having a minimum value equal to or greater than 1.0 and a maximum value equal to or less than 10.0, such as, for example, 2.4 to 7.6. Any maximum numerical limitation recited in this specification is intended to include all lower numerical limitations subsumed therein and any minimum numerical limitation recited in this specification is intended to include all higher numerical limitations subsumed therein. Accordingly, Applicant reserves the light to amend this specification, including the claims, to expressly recite any sub-range subsumed within the ranges expressly recited herein. All such ranges are intended to be inherently described in this specification such that amending to expressly recite any such sub-ranges would comply with the applicable disclosure requirements.

"Bound," "bind", "binding", "associated with", or "attachment", "attached to" and the like as used herein with repsect to the tetraamido macrocyclic ligand activator and the carbon support means covalent or non-covalent binding, including without limitation, the attractive intermolecular forces between two or more compounds, substituents, molecules, ions or atoms that may or may not involve sharing or donating electrons. Non-covalent interactions may include ionic bonds, hydrophobic interactions, hydrogen bonds, van der Waals forces (dispersion attractions, dipole-dipole and dipole-induced dipole interactions), intercalation, entropic forces, and chemical polarity.

"Carbon-containing" as used herein means that the support contains a sufficient amount of carbon to bind at least one molecule of a tetraamido macrocyclic metal ligand described herein. Exemplary carbon sources include activated carbon, amorphous carbon, graphite, charcoal, and carbon-rich compositions.

"Carbon rich" as used herein means that the dominant component in the composition, solution, or mixture, is carbon. Other components may be present in minor amounts relative to the amount of carbon present.

Macrocyclic tetradentate metal ligands useful as the catalytic activators in the present invention have the general structure 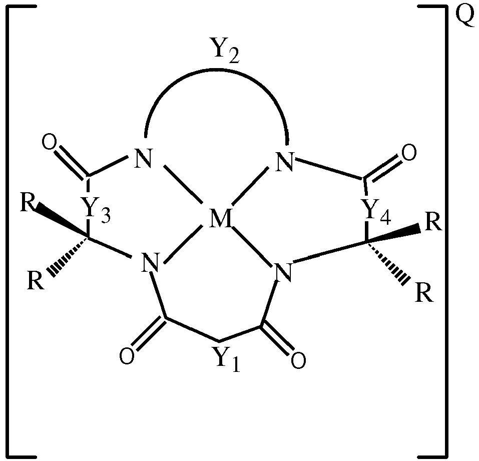 wherein Y₁, Y₃ and Y₄ each represents a bridging group, having zero, one, two or three carbon containing nodes for substitution, and Y₂ is a bridging group having at least one carbon containing node for substitution, each said node containing a C(R) or a C(R)₂ unit and each R substituent is the same or different from the remaining R substituents and ( i ) is selected from the group consisting of alkyl, cycloalkyl, cycloalkenyl, alkenyl, aryl, alkynyl, alkylaryl, halogen, alkoxy, or phenoxy, CH₂CF₃, CF₃ and combinations thereof, or (ii) form a substituted or unsubstituted benzene ring of which two carbon atoms in the ring form nodes in the Y unit, or (iii) together with a paired R substituent bound to the same carbon atom form a cycloalkyl or a cycloalkenyl ring, which may include an atom other than carbon, e.g., cyclopropyl, cyclobutyl, cyclopentyl, or a cyclohexyl ring; M is a transition metal with oxidation states of I, II, III, IV, V, VI, VII or VIII or selected from Groups 3, 4, 5, 6, 7, 8, 9, 10, and 11 of the Periodic Table of the Elements; and, Q is any counterion which would balance the charge of the compound on a stoichiometric basis.

The carbon supported tetradentate macrocyclic metal ligands are useful as oxidatively stable and surprisingly robust oxidant activators in combination with an amount of a source of an oxidant effective for oxidizing a target oxidizable substrate or compound.

Various methods for making the embodiments of the tetradentate macrocyclic ligands encompassed by the general structure above, further details about the macrocyclic ligands, and uses therefor are disclosed in the following U.S. patents and therefore, that information need not be repeated here: U.S. Patents Nos. 5, 847,120, 5,853,428, 5,876,625, 6,011,152, 6,051,704, 6,054,580, 6,099,586, 6,100,394, 6,136,223, 6,241,779, 6,992,184, and 7,060,818, the disclosures of each of which is hereby incorporated herein by reference.

The oxidation may be for the purpose of oxidizing any oxidizable compound in an aqueous media, such as water or a vapor, to remove the oxidizable target compound or substrate from the aqueous media or to degrade the oxidizable target compound or substrate to harmless or less harmful forms. The oxidizable compound or substrate may include any oxidizable compound from any source found in water, such as aromatic groups, conjugated pi systems, dyes, colorants, pharmaceuticals, estrogens, androgens, endocrine disrupting compounds, carcinogens or suspected cancer-causing agents, certain personal care products, food additives, food products, natural organic matter, species arising from industrial processes, species found in municipal or industrial waste waters, such as products and byproducts of the synthetic chemicals, natural gas and oil, nuclear energy agricultural, pesticides, plastics, pulp and paper, printing, or defense industries, medical waste, oxidizable pathogens or undesirable living organisms found in water.

The tetraamido macrocyclic ligand activators have been successfully used with hydrogen peroxide to rapidly deactivate bacterial spores, rendering them incapable of germination and reproduction. *See* "Green" Oxidant Catalysts for Rapid Deactivation of bacterial Spores, Angew. Chem. Int. Ed., vol. 45, pp.3974-3977 (2006).

Those skilled in the art will recognize that the effectiveness of the carbon supported tetraamido macrocyclic ligand catalytic activators described herein with respect to any given target contaminant will vary for different formulations of the activator and different carbon support sources. Some ligand formulations on certain carbon supports will more effectively (*i.e.,* more thoroughly or more quickly) remove some contaminants than other contaminants. The nature of the contaminant to be removed or degraded and the environment in which it is found may dictate which formulation of the ligand activator and carbon support is used.

The tetraamido macrocyclic metal ligand complexes are useful oxidant activators. Of these, those having a substituted aromatic substituent fused directly into the ligand's cyclic structure are especially preferred.

For example, an exemplary useful compound has the structure:

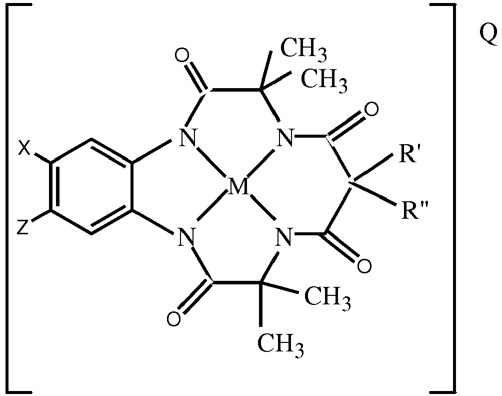

wherein X and Z may be H, electron-donating or electron-withdrawing groups and R' and R" may be any combination of H, alkyl, cycloalkyl, cycloalkenyl, alkenyl, aryl, alkynyl, alkylaryl, halogen, alkoxy, or phenoxy substituents, or combine to form a cycloalkyl or cycloalkenyl ring, which may contain at least one atom that is not carbon; M is a transition metal with oxidation states of I, II, III, IV, V, VI, VII or VIII or selected from Groups 3, 4, 5, 6, 7, 8, 9, 10, and 11 of the Periodic Table of the Elements; and Q is any counterion which would balance the charge of the compound on a stoichiometric basis.

The counterion may be selected from the group consisting of tetraarylphosphonium ions, tetraalkylammonium ions, and bis- (triphenylphosphorananylidene)-ammonium ions for balancing the charge of the compound on a stoichiometric basis. The tetraarylphosphonium ions include, for example, tetraphenylphosphonium ions+ or a related ion with four aryl or four alkyl groups on phosphorus including with any combination of mixed aryl or alkyl, or mixed aryl and alkyl groups on the phosphorus atom in the one ion. The tetraalkylammonium ions may include, for example, tetraethyl ammonium+, tetrapropyl ammonium+, and tetrabutyl ammonium+ ions, and tetralkyl ammonium ions with longer straight chain groups including ions with mixed straight chain alkyl groups on the nitrogen atom in the one ion, or with four branched alkyl groups or with mixtures of branched and straight chain alkyl groups on the nitrogen atom in the one ion.

In certain embodiments, the method for using the carbon supported tetraamido macrocyclic ligand catalytic activator comprises generally, the steps of contacting an aqueous fluid in need of cleansing, or an effluent stream in need of cleansing, with a source of an oxidant, preferably a peroxy compound, and more preferably hydrogen peroxide and/or its dissociation products, and one or more carbon supports having catalytic, or substoichiometric, amounts of the tetraamido macrocyclic ligand activator bound or attached to the support.

The method may be run at a variety of temperatures, but preferably within the range of ambient to about 130°C, and more preferably between ambient to 90°C. Ranges of ambient to about 40°C may also be successfully used. Temperature, however, does not appear to be critical. A wide range of temperatures are suitable.

The pH may be neutral to basic. The preferred pH range is between 7 and 12, and preferably between 9 and 11, and most preferably under basic conditions at or near pH 10.

While the carbon supported activator of the present invention has been shown in other applications to be an excellent activator for oxidation reactions in solution in general, and particularly as an activator for activating O-atom transfer oxidants, such as hydrogen peroxide, t-butyl hydroperoxide, cumyl hydroperoxide, hypochlorite and peracids, the preferred use in the method of the present invention is as an activator of peroxy compounds, and most preferably as an activator of hydrogen peroxide in aqueous fluids, such as liquid water or water vapor.

The unexpected finding that the binding of tetraamido macrocyclic metal ligands on solid carbon-containing supports does not diminish the catalytic activity of the ligand activators, and to the contrary, surprisingly results in robust, long acting reusable activators are believed to be an important step in advancing industrial applications for the family of tetraamido macrocyclic ligand activators. The ability of the carbon supported tetraamido macrocyclic ligand activators to oxidize oxidizable compounds makes them useful for removing persistent organic pollutants, or removing from water any of the oxidizable target substrates or compounds described above. In the absence of the attachment to a support described herein, it has heretofore been found that too much catalyst is required for large scale or commercial scale uses to be cost-effective. Only the most highly polluted waters justified the cost of prior tetraamido macrocyclic ligands to catalyze oxidation and removal of organic pollutants.

Various embodiments of the method of the invention use a solid carbon-containing support to bind or attach to an embodiment of tetraamido macrocyclic metal ligands having organic cations, such as tetraphenylphosphonium. In this embodiment of the tetraamido macrocyclic ligand, the sodium counterion used predominantly in an earlier water-soluble embodiment of the ligand is replaced with an organic cation. Certain embodiments of the Fe-tetraamido macrocyclic ligands having organic cations can be loaded onto the carbon containing support, such as activated carbon, in common solvents (*e.g.,* water or organic solvents, such as methanol). Surprisingly, the carbon supported ligand catalysts have been found to provide sustained activity over significantly longer periods of time than heretofore thought possible, and, were further found to be re-usable multiple times. In at least one embodiment, the carbon supported ligand catalytic activators provided sustained activity for at least one week during which time the carbon supported catalyst were used nine times for water treatment. The initial rate is greatest with the first use and then slows with additional use cycles to reach a point of no further, or very slow, change. Although the catalytic activity decreases over time, it appears that the catalytic activity in certain embodiments was reduced no more than 50% after one week.

Importantly, the initial rate of oxidation dye found for this relatively stable activity regime is comparable to the initial rate found for the homogeneous version of the catalyst and the dye bleaching is occurring without stirring. This means that reactors that increase the efficiency of dye contact with the solid supported catalysts should turn in much higher initial rates.

The carbon supported tetraamido macrocyclic ligand catalytic are useful to catalyze the activity of a variety of oxidants. For environmental considerations, the oxidant for removal of certain contaminants from water is preferably a peroxy compound or ozone. Previous attempts to use ferrous ion catalyzed peroxide treatment for removal of absorbable organic halogen have proven to be unfeasible due to the very high levels of peroxide needed and the prohibitive expense associated with the use of large quantities of peroxide. The addition of the carbon supported activator compound of the present invention to peroxy compounds has been demonstrated to significantly lower the level of peroxide needed for oxidation reactions. Thus, the carbon supported activator/oxidant composition of the present invention is believed to be well suited to the removal or neutralization of organic pollutants, such as dyes, organochlorine and recalcitrant carbonaceous materials. Moreover, the ability to reuse the supported catalytic activators reduces significantly the amount of the catalytic activator needed and therefore, the cost of the oxidation system.

The family of tetraamido macrocyclic ligand catalytic activators has been shown to be highly effective at oxidizing aromatic and other conjugated species. Detailed mechanistic studies have been performed to elucidate the pathways in peroxide activation and subsequent oxidation of substrates, and representative reactions are shown below.

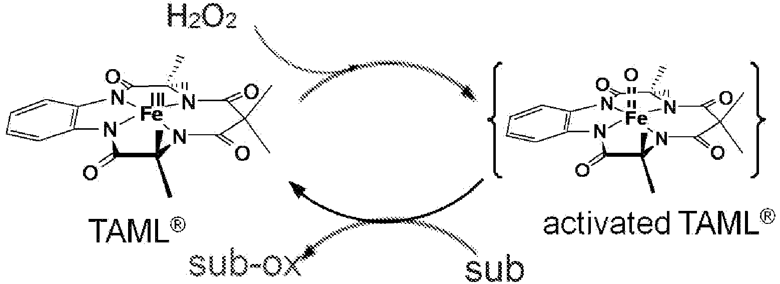

The schematic shows the activation of a commercially available form of the tetraamido macrocyclic ligand catalytic activators (TAML^{®}, sold by GreenOx Corp., Pittsburgh, Pennsylvania) upon the addition of hydrogen peroxide and the oxidation of a substrate. "Sub" as used in the above schematic refers to the substrate to be oxidized, such as a organic pollutants and pathogens. "Sub-ox" refers to the oxidized substrate. The schematic below shows an exemplary contaminant, yellow dye #5, contacted by the activated oxidant to yield any one or more of the oxidized substrate degradation constituents.

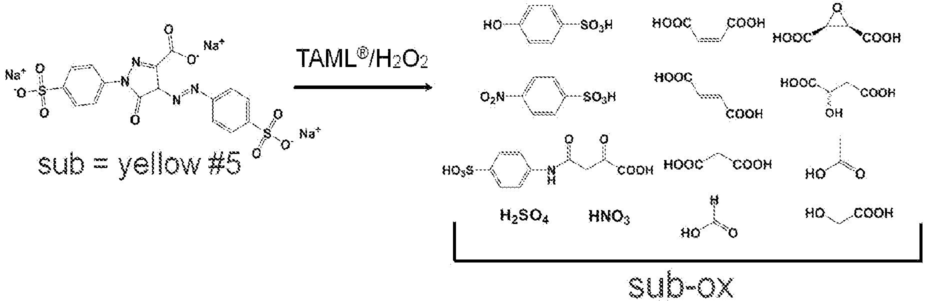

Application of the carbon supported activator/oxidant system of the present invention at low level wastewater streams, either at the discharge site or a recycle site, are believed to be advantageous. Treatment of combined wastewaters at end-of-pipe sites have the advantage of having had the majority of degradable organic material in the effluent removed, leaving only the compounds and materials that have escaped the existing battery of cleaning technologies to be targeted by the carbon supported activator/oxidant system of the invention. Treatment at an earlier stage upstream of the end-of pipe site has the advantage of being able to expose a greater concentration of target oxidizable compounds to the carbon supported activator/oxidant system of the invention. In certain embodiments, the carbon supported catalytic activator may be housed in or on one or more replaceable filter surfaces or in one or more replaceable filter cartridges positioned in one or more locations along an effluent stream or in a pipe-line. The contaminant laden aqueous media flows through the filter housings and is exposed to the supported catalytic activators whereby the oxidizable contaminants are oxidized to a non-hazardous or less hazardous form.

The amount of tetraamido macrocyclic metal-ligand added to the carbon in general has a mass fraction less than one. The amount of the supported complex added to solution may vary depending on the intended use. As the inventive carbon supported tetraamido macrocyclic metal-ligand complexes act in a catalytic fashion, the amount thereof added to the oxidant is generally substoichiometric.

### Oxidant Compounds

The oxidant compounds, such as O transfer atoms, preferably peroxy compounds, can be an organic or inorganic compound containing the -O-O-peroxide linkage. Exemplary compounds include hydrogen peroxide, hydrogen peroxide adducts, t-butyl hydroperoxide, cumyl hydroperoxide, hypochlorite and peracids, compounds capable of producing hydrogen peroxide in aqueous solution, organic peroxides, persulfates, perphosphates, and persilicates. Hydrogen peroxide adducts include alkali metal (*e.g.,* sodium, lithium, potassium) carbonate peroxyhydrate and urea peroxide which may liberate hydrogen peroxide in solution. Compounds capable of producing hydrogen peroxide in aqueous solution include alkali metal (sodium, potassium, lithium) perborate (mono-and tetrahydrate). The perborates are commercially available from such sources as Akzo N.V., and FMC Corporation. Alternatively, an alcohol oxidase enzyme and it appropriate alcohol substrate can be used as a hydrogen peroxide source. Organic peroxides include, without limitation, benzoyl and cumene hydroperoxides. Persulfates include potassium peroxymonosulfate (sold as Oxone®, E.I. duPont de Nemours) and Caro's acid. More generally, oxidizing compounds such as chlorine, bromine, chlorine dioxide, ozone, oxygen, radical oxidants produced photochemically, permanganate, ferrate, cerium ion, or other oxidizing entities in water are subject to activation by the compositions of this invention.

Tetraamido macrocyclic metal ligand catalytic adsorption onto carbon supports has been performed from water and from organic solvents. By recovering residual metal ligand catalytic activator from the flask in which adsorption was performed, it was possible to assess the fraction that adheres to the carbon support.

Adsorption of the tetraamido macrocyclic metal ligand catalytic activators onto or within carbon surfaces may be accomplished by dissolving the catalyst in an organic solvent. In embodiments of the method where the solvent is removed by evaporation, volatile organic solvents are the solvent of choice. Examples of commonly used solvents include methanol, ethanol, methylene chloride, and chloroform, but those skilled in the art will recognize that other organic solvents may be used. The carbon substrate is submerged in the solvent containing the catalyst and the solvent is removed, for example, through evaporation, by placing the mixture under vacuum, or by solvent exchange. A fraction of the catalyst remains on or within the carbon support when placed in an aqueous environment or a liquid that is a non-solvent for the particular form of the catalyst, such as hydrocarbons. For example, in certain embodiments, fractions of the oxidant activator up to and including 100% will bind to the carbon support. Under the conditions described herein, fractions from 30% to 100% of the oxidant activator have been bound to the carbon-containing support to form a supported catalytic activator. A fraction of tetraamido macrocyclic ligand catalyst that remains on or within the carbon support is catalytically active and is capable of reacting with the oxidant of choice for oxidizing oxidizable target substrates.

Tetraamido macrocyclic metal ligand catalytic activators may also be associated with the carbon supports directly from water by attachment to carbon supports with higher affinities for organic molecules. In this preparation method, the carbon support can be placed in aqueous media containing the catalyst and the catalyst adsorbs on or within the carbon support. Under these circumstances, it is generally not necessary to remove the solvent from the system, and oxidation reactions may be performed following binding of the activators to the carbon support in the aqueous medium.

### EXAMPLES

Experiments are presented herein which demonstrate the usefulness of the supported catalytic activator composition for the present invention.

Examples showing the preparation and use of exemplary tetraamido macrocyclic metal ligand catalysts bound to a diversity of carbon supports follow.

Several members of the family of tetraamido macrocyclic metal ligand catalytic were tested. Tetraamido macrocyclic ligand catalysts activators, referred to as FeB*, are pentacoordinated species, usually with an axial aqua ligand, in the solid state. Members of this group of catalysts are collectively referred to as FeB*. The aqua complexes are synthesized as such or as the corresponding chloro species with Cl⁻ instead of H₂O (and with two as opposed to one M⁺ = Li⁺, Na⁺, NR₄⁺, etc. counter ions). A representative structure for FeB* is shown below, with the exemplary variations in substituent groups set forth in Table 1.

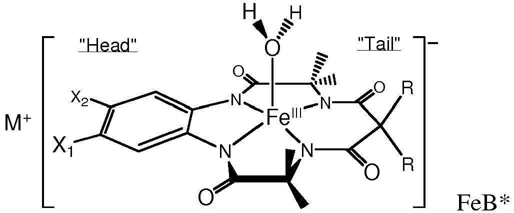

**Table 1.**

| **FeB*** | X₁ | X₂ | R |
|---|---|---|---|
| **a** | H | H | Me |
| **b** | Me | Me | Me |
| **c** | Me | H | Me |
| **d** | MeO | MeO | Me |
| **e** | NO₂ | H | Me |
| **f** | COOMe | H | Me |
| **g** | COOH | H | Me |
| **h** | CONH(CH₂)₂NMe₃⁺ | H | Me |
| **i** | Cl | Cl | Me |
| **j** | Cl | Cl | Et |
| **k** | Cl | Cl | F |
| **l** | H | H | F |
| **m** | H | H | 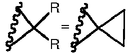 |
| **n** | Cl | Cl | 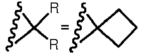 |

Tetraamido macrocyclic metal ligand catalytic activators, referred to herein as FeD*, are usually pentacoordinated species, usually with an axial aqua ligand (L) in the solid state, but may be hexacoordinated with any combination of two suitable axial ligands (L) with the two being the same or different and usually with iron (Fe) as the central metal (M), but may be any transition metal in the central site. The aqua complexes are synthesized as such or as the corresponding chloro species with Cl⁻ instead of H₂O as axial ligand in a five-coordinate species (and with two as opposed to one C = Li⁺, Na⁺, NR₄⁺, etc. counter ions). For use herein, FeD* represents the following ligand catalytic activator, with the variations in structure shown in Table 2.

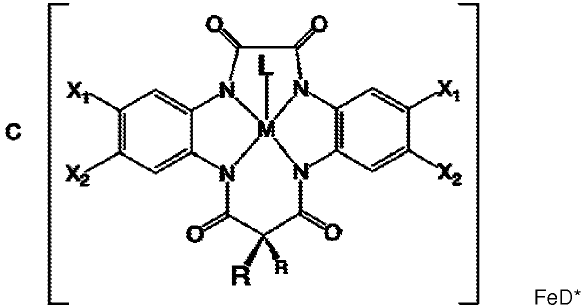

**Table 2**

| FeD* | X₁ | X₂ | R |
|---|---|---|---|
| a | H | H | Me |
| b | NO₂ | H | Me |
| c | Cl | Cl | Me |

### Chemicals

The catalysts tested include variations of the FeB*, FeBcp, and FeD* forms of the activators, shown below, which were prepared using standard synthesis and purification methods (TAML oxidant activators: A new approach to the activation of hydrogen peroxide for environmentally significant problems. T. J. Collins, Acc. Chem. Res., 2002, 35, 782-790.). Organic salts for replacing Na⁺ in the FeB* catalysts included tetraphenylphosphonium chloride (PPh₄Cl), tetrabutylammonium bromide (Bu₄NBr), and bis-(triphenylphosphorananylidene)-ammonium chloride (PNPCl) were obtained from Sigma-Aldrich, Inc. (St. Louis, MO). Orange (II) dye [abbreviated Or(II)] was also purchased from Sigma-Aldrich. Trichlorophenol was obtained from Sigma-Aldrich; St. Louis, MO. American Chemical Society (ACS)-grade K₂HPO₄ was purchased from EMD Chemicals Inc. (Gibbstown, NJ) and KH₂PO₄ was obtained from Acros Organics (Geel, Belgium) which were used to make the phosphate buffer, and 30% H₂O₂ was obtained from Fisher Scientific (Pittsburgh, PA). All solvents used were high performance liquid chromatography (HPLC) grade and obtained from Fisher Scientific.

The structures for some of the tetraamido macrocyclic ligand catalytic activators tested are shown below.

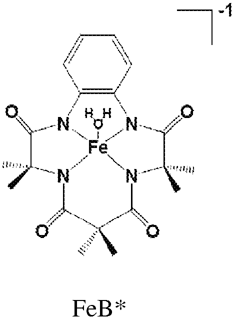

FeB* is used herein as a convenient shorthand for ferrate(1-),[3,4,8,9-tetrahydro-3,3,6,6,9,9-hexamethyl-1H-1,4,8,11-benzotetracyclotridecine-2,5,7,10(6H,11H)-tetronato(4-)-kappa.N1,kappa.N4,kappa.N8,kappa.N11].

An alternative form of FeB* is shown below.

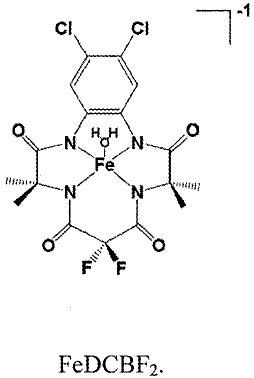

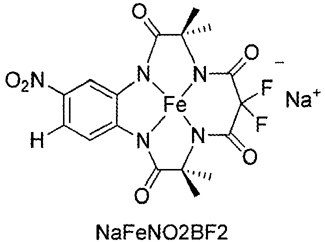

Na FeNO₂BF₂ is used herein as a convenient shorthand for ferrate(1-),[3,4,8,9-tetrahydro-6,6-difluoro-3,3,9,9-tetraamethyl-13-nitro-1H-1,4,8,11-benzotetracyclotridecine-2,5,7,10(6H,11H)-tetronato(4-)-kappa.N1,kappa.N4,kappa.N8,kappa.N11]-,sodium.

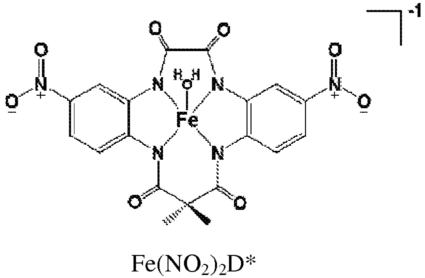

FeD* is used herein as a convenient shorthand for ferrate(1-),[15,15-dimethyl-5H-1,4,7,11-dibenzotetracyclotridecine-6,7,14,16(8H,13H,15H,17H)-tetronato(4-)-kappa.N1,kappa.N4,kappa.N7,kappa.N11.

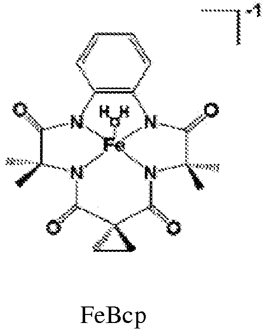

FeBcp is used herein as a convenient shorthand for ferrate(1-),[3,4,8,9-tetrahydrospiro3,3,9,9-tetraamethyl-6,1'-cyclopropane-1,4,8,11-benzotetracyclotridecine-2,5,7,10(1H,11H)-tetronato(4-)-kappa.N1,kappa.N4,kappa.N8,kappa.N11].

A diversity of carbon supports were used for binding the Fe-tetraamido macrocyclic ligand catalytic activators: OLC™ 12 x 30 granular activated carbon was obtained from Calgon Carbon Corporation (Pittsburgh, PA). HP-120 powdered activated carbon was obtained from Pica (Saint-Maurice Cedex, France). C-NERGY™ synthetic graphite was obtained from Timcal Graphite & Carbon (Bodio, Switzerland).

### Measuremertt of tetraamido macrocyclic ligand catalysts and Or(II) solution concentration

The concentration of tetraamido macrocyclic ligand catalysts in aqueous or non-aqueous media can be determined by spectrophotometric techniques involving the absorption of optical light. The B* analogue has an absorption maximum that depends on the solvent used, generally close to 365 nm, and solution concentration can be determined by measuring absorption at this wavelength and comparing the measured value to calibration curves. The concentration of Or(II) solutions can also be determined using spectrophotometric techniques; Or(II) has an absorption maximum near 485 nm.

Spectrophotometric measurements were performed using a Hewlett-Packard Diode Array spectrophotometer model 8453 (Palo Alto, California) equipped with a thermostated cell holder and an automatic 8-cell positioner. Temperature was controlled by Thermo digital temperature controller RTE17 with an accuracy of ±1 °C.

### Tetraamido macrocyclic ligand catalytic activator attachment

For binding from methanol, methylene chloride, 1 x 10⁻⁵ moles of tetraamido macrocyclic ligand catalyst were added to a 20 mL glass vial and dissolved in 1.5 mL solvent. Then 450 mg of carbon were added to the solution and 1 mL solvent was used to rinse off the side of the vial. Vacuum was then applied to remove the solvent and dried, catalyst-loaded carbon can be placed in solution in which oxidation reactions can be performed. Those skilled in the art will understand that other organic solvents may be used as long as the tetraamido macrocyclic ligand catalyst dissolves in the solvent. Volatile solvents would be useful where the solvent is to be evaporated.

For binding from aqueous solutions, 1 x 10⁻⁵ moles of tetraamido macrocyclic ligand catalyst were added to 1.5 mL water. Then 50 - 150 mg of carbon were added, which generally resulted in the formation of a colorless solution containing the carbon particles. The catalyst-loaded carbon could then be recovered via filtration or, alternatively, peroxide and oxidation substrate, such as Or(II) dye, could be added directly to the solution to test reactivity.

### Scanning Electron Microscopy

The samples were mounted on a glass slide and coated with gold before observation using the scanning electron microscope. The scanning electron microscope used was a Hitachi S-2460N.

### Determination of degree of adsorption onto activated carbon

Carbon-containing tetraamido macrocyclic metal ligand catalyst was removed from each vial used during the preparation, and 2.0 mL methanol was added to each vial. Then 100 µL of this solution were added to 1.9 mL methanol in a quartz cuvette and UV-Vis measurements of each methanol solution were then taken. The absorbance at 375 nm was measured for methanol solutions. A calibration curve of PPh4FeB* in methanol from 0 to 6.5 x 10⁻⁵ M was also generated in order to determine an extinction coefficient (3874.3 M-¹cm⁻¹) to calculate the moles of Fe-tetraamido macrocyclic ligand catalysts remaining in each vial. Similar extinction coefficients were determined for PPh4FeBcp (7266 M⁻¹cm⁻¹) and NaFeB* (3704 M⁻¹cm⁻¹). Extinction coefficients were determined for the Bu4NFeB* (4854 M⁻¹cm⁻¹), and PNPFeB* (5465 M⁻¹cm⁻¹) that were prepared. For NaFeB* in buffer the maximum absorbance shifts to 365 nm. Other members of the tetraamido macrocyclic ligand catalyst family had similar molar absorptivity values. See for example, the family members listed in Tables 1 and 2. The amount of catalyst that was not associated with the carbon was determined by adding a known volume of water to dissolve residual catalyst and measuring the optical absorption value at 365 nm.

### Pre-saturation with Or(II)

In a standard measurement, 150 mg of carbon was placed into a 20 mL glass vial and put under vacuum after which a 3 mL 5.17 x 10⁻³ M tetraamido macrocyclic ligand catalyst methanol solution was added for the solvent-removal method. Three test runs of 50 mg samples of each type of carbon were first saturated with 2.0 mL of 2.7 x 10⁻³ M Or(II) solution for up to 2 days (i) to avoid competing adsorption of Or(II) onto the carbon supports and (ii) to ensure that loss of Or(II) color was not due to adsorption to the carbon and (iii) to ensure that oxidation was the dominant mode through which the absorption of optical light by Or(II) decays with time during the experiments. These catalyst-loaded carbon samples were then removed from their saturation vials and used for bleaching runs reported here.

### Typical bleaching reactions

Reaction conditions were typically pH 7 (0.01 M phosphate buffer), 4 x 10⁻⁵ M [Or(II)], 0.0023 M [H₂O₂] at 25 °C. In these experiments, 45 µL of 2.7 x 10⁻³ M Or(II) stock solution and 60 µL of 0.115 M H₂O₂ solution were added to reaction vessels containing tetraamido macrocyclic ligand catalyst. Reactions were carried out in 1 mL cuvettes or in glass vials. Granular activated carbon is comprised of larger particles, which generally settled to the bottom of the vial, while powdered activated carbon is composed of sub-millimeter particles that were suspended in solution. Bleaching of Or(II) dye can be determined using spectrophotometric techniques. Bleaching of the dye by the catalyst resulted in a monotonic decrease in optical absorbance at 485 nm as a function of time.

### Oxidation of Chlorophenols

It has been shown that the tetraamido macrocyclic ligand catalytic activators and hydrogen peroxide oxidize pollutants, such as chlorophenols. *See* Gupta, S. S., M. Stadler, C. A. Noser, A. Ghosh, B. Steinhoff, D. Lenoir, C. P. Horwitz, K.-W. Schramm, T. J. Collins, Rapid total destruction of chlorophenol pollutants by activated hydrogen peroxide, Science, 2002, 296, pp. 326-328. The following experiments show that the ability of the tetraamido macrocyclic ligand catalytic activators to oxidize chlorophenol pollutants is not diminished in the carbon supported form described herein.

To test the effectiveness in oxidizing other organic species in solution, 2 mg of the FeNO₂BF₂ activator were dissolved in 1 mL water and then 50 mg of powdered activated carbon were added. Following similar procedures as described in the reactions with Or(II), 19 mL of 45 µM trichlorophenol (Sigma-Aldrich; St. Louis, MO) were added. Subsequent to this, 20 µL of 30% hydrogen peroxide solution were added and the reaction was allowed to occur for 450 seconds. The carbon was removed by filtration and the absorbance value at 316 nm was measured to determine the change in trichlorophenol concentration compared to carbon-containing solution that lacked the oxidant activator. Under these conditions, at 15% reduction in trichlorophenol concentration were measured, indicating that this system is capable of oxidizing the same range of species as the homogeneous activators.

### Measurement of phosphate-mediated demetallation

In a sample experiment, fifteen 150 mg samples of NaFeB*-loaded carbon were allowed to sit in 2 mL of 0.01 M phosphate buffer. At the same time, three vials of a 2.03 x 10⁻⁴ M NaFeB* in 0.01 M phosphate buffer stock solution were prepared. Three catalyst-loaded carbon samples were removed from the buffer 1 day after immersion in the phosphate buffer by filtration and the NaFeB* was desorbed from the carbon by placing the carbon in methanol solution for 15 min. The experiment was repeated daily for 5 days. Absorbance measurements of the methanol solutions were then taken at 375 nm, by removing 100 µL of and diluting to 2.0 mL. In addition, bleaching runs were then carried out with the homogeneous NaFeB* in phosphate buffer samples at the same time as the extracted NaFeB* samples to compare catalyst activity. The same experiment was carried out in 0.01 M phosphate buffer and 0.1 M phosphate buffer.

Binding onto powdered activated carbon (Pica) was tested for FeB* and FeD* from both organic solvent through catalyst dissolution in methanol followed by solvent removal under vacuum as well as direct attachment from water. In the FeB* experiments, 5 mg of catalyst were adsorbed to 150 mg of carbon while in the FeD* experiments, 2 mg of catalyst were adsorbed to 50 mg of carbon. Differences between the extent of bleaching between FeB* and FeD* in these experiments may be attributed to differences in catalyst concentration.

| Catalyst | Solvent | % Or(II) Bleached |
|---|---|---|
| FeB* | water | 81% |
| FeB* | methanol | 39% |
| FeD* | water | 70% |
| FeD* | methanol | 62% |

These results indicate that binding from water and organic solvent are both viable deposition strategies for this powdered activated carbon, but aqueous deposition may result in more active catalyst. This may be due to the drying step involved in the organic solvent method, which could alter the structure and porosity of the activated carbon.

Binding onto graphite powder was also attempted. Direct adsorption from aqueous solution was less effective. Graphite powder did not mix readily into aqueous solutions of FeB* and less than 10% of the catalyst remained associated with the graphite following repeated washing in water and less than 5% of Or(II) bleached in subsequent reactions. Deposition by organic solvent vacuum removal was performed using methanol to compare loading strategies. In this case, 59% of the FeB* in the original methanol solution remained on the graphite following vacuum removal of the methanol and repeated washing with water. However, the catalyst appeared to be less active on the graphite, with 13% of Or(II) being bleached in subsequent oxidation reactions. These results indicate that loading tetraamido macrocyclic ligand catalysts onto graphite is feasible but the resulting supported catalyst may be less active in bleaching reactions than on other types of carbon.

Loading efficiency of different types of catalyst were tested as a function of catalyst type, counterion, and carbon type by measuring the optical absorbance of the residual solvent following exposure to the carbon. Bleaching effectiveness of the catalyst-loaded carbons were determined by measuring the concentration of Or(II) remaining after 450 seconds (represented in units of molarity or M) or the percent decrease of Or(II) dye following reaction for 450 seconds under static conditions was used as a metric to assess activity of a given formulation. An alternative metric was to measure the initial rate of decrease of the absorbance of the Or(II) solution in the reaction; this metric has units of M⁻¹s⁻¹.

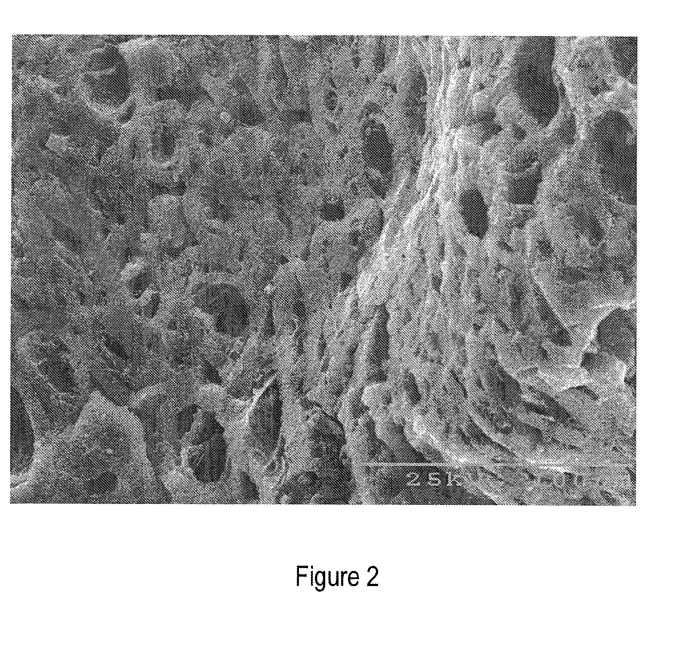

On granular activated carbon (obtained from Calgon Carbon, Inc.), loading efficiency of different salts of FeB* were compared. Figure 2 shows the granular activated carbon after the NaFeB* catalytic activator was bound to the carbon by methanol. The carbon remained porous after the solvent processing. The average loading efficiency of NaFeB* was 68% while PPh₄FeB* had an average loading efficiency of 74%, PNPFeB* was 87%, and Bu₄NFeB* was 88%. Similar loading levels were obtained for FeBcp, suggesting that other members of the family have similar adsorption characteristics. From these results, it may be concluded that organic counterions may improve catalyst affinity for carbon surfaces over analogues that contain the sodium counterion.

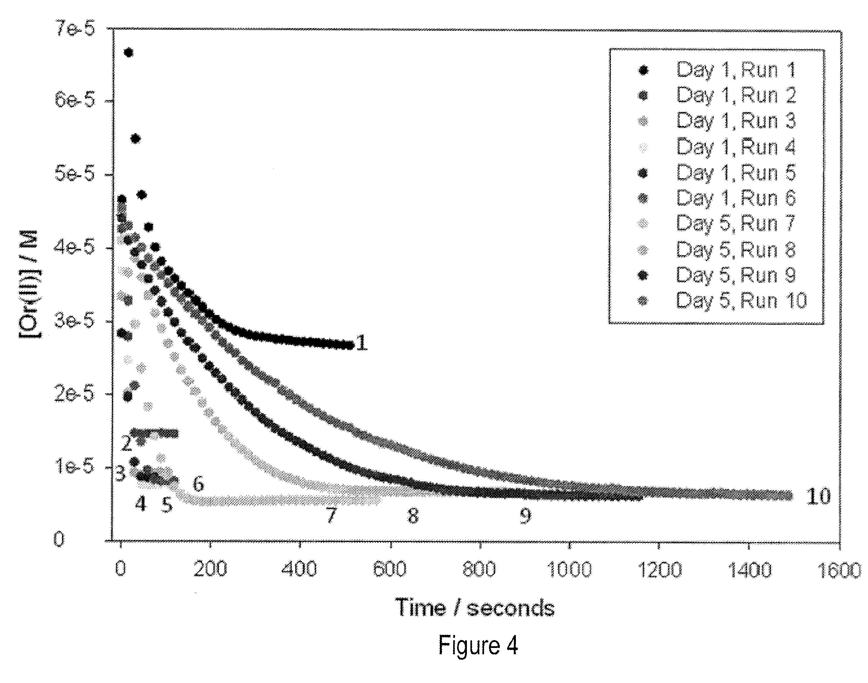

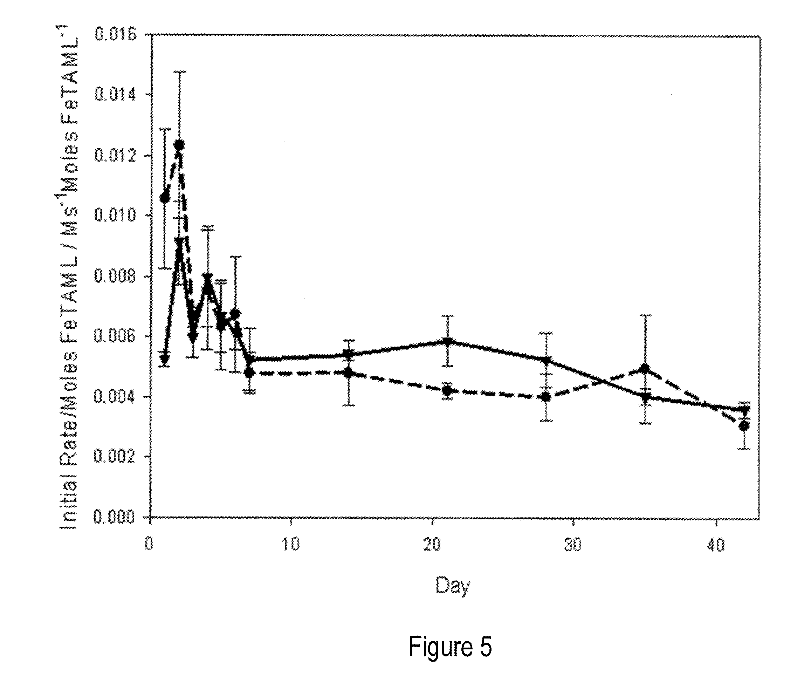

The bleaching efficiency of these formulations on granular activated carbon were measured. Representative bleaching traces of FeB* are shown in Figure 4. All curves were well fit by single exponential decays. Some variability in the kinetics was observed between runs, but the carbon supported catalytic activators all appeared to be active over numerous runs spanning several days. The robustness of the carbon supported catalytic activator systems are shown in Figure 5 in which oxidation runs were performed over a period of 40 days for NaFeB* and PPh4FeB*. Both showed active oxidation of Or(II) over this extended period. Similar results were obtained for the FeBcp analogue, demonstrating that binding of the tetraamido macrocyclic ligand catalytic activators to the carbon-containing support is effective across counterions and individual members of the catalyst family and that the supported catalytic activators described herein may be reused multiple times for periods of time sufficient to oxidize oxidizable substrates of interest. Those skilled in the art will appreciate that the supported catalytic activators may be reused for as long as it takes, under the conditions of use, to deactivate the catalytic activator or foul the carbon-containing support. When deactivated, there will be a measurable decrease in oxidation, at which time, if continued removal of contaminants is desired, the one or more supported macrocyclic catalytic activators in the aqueous media can be replaced with fresh supported macrocyclic catalytic activators.

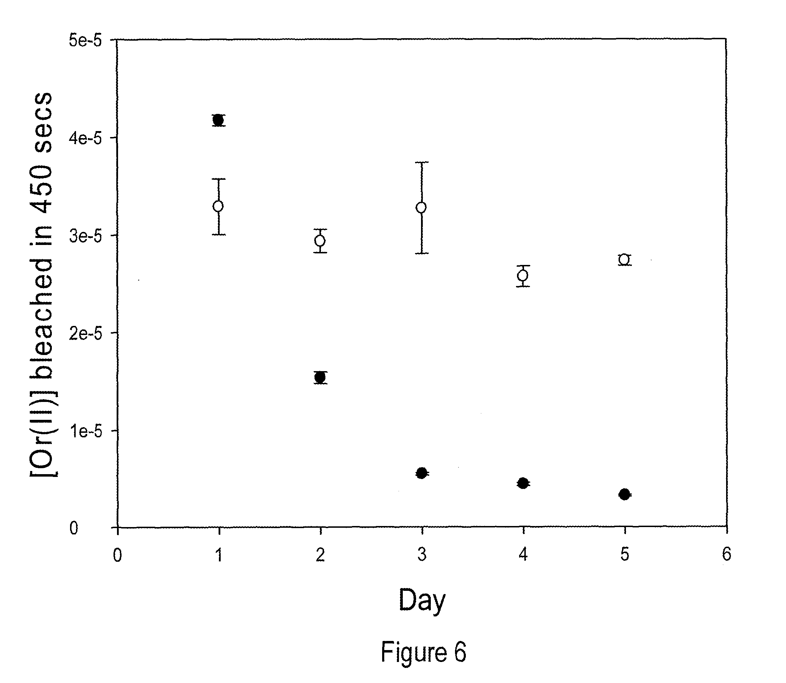

Resistance to phosphate-mediated demetallation was also tested. In 0.1 M phosphate solution, FeB* loses activity within 5 days due to abstraction of the Fe³⁺ ion. To determine resistance to demetallation of FeB* bound to granular activated carbon, catalyst-loaded carbon was placed in 0.1 M phosphate solution for up to 5 days. Each day a sample was removed from phosphate solution and placed in methanol, which removed the adsorbed catalyst from the surface. The concentration and activity of the removed FeB* was compared to FeB* in phosphate buffer, and the results are shown in Figure 6. The catalyst that was loaded on carbon retained its activity while the homogeneous catalyst was essentially deactivated after 5 days. These results suggest that binding may enhance catalyst lifetime, even against deactivating reactions.

Other forms of carbon supports may be used for this application. For example, carbon black is considered to be a partially amorphous carbon that can be an effective sorbent of organic matter and may offer an alternative to types of activated carbon.

In an alternate preparation strategy, vapor-phase deposition of catalyst is feasible by forming an aerosol of the catalyst in water or non-aqueous solvent. This can be accomplished using accepted methods for aerosol formation. For example, delivery from a pressurized vessel through a small nozzle is a common method for preparing aerosol sprays that could be used to deliver aqueous or non-aqueous solutions containing the oxidant activator. (*See* for example, Aerosols: Science and Technology. I. Agranovski, ed. 2010; Wiley-VCH; ISBN-10: 352732660X) In this case, the spray may be directed at the carbon support to allow deposition on the surface following transfer from the vapor phase to achieve similar catalyst loading levels as described in this application. Other strategies based on boiling the solvent or otherwise produce a gaseous dispersion are also possible.

An alternate use of the catalyst is in the removal of contaminants from vapors formed through boiling, aerosol formation, or other means of vaporization. An example of this approach is oxidation of organic contaminants in water vapor from gas streams. (*See* for example, Removal of VOCs from humidified gas streams using activated carbon cloth, Mark P. Cala, Mark J. Rood and Susan M. Larson, Gas Separation & Purification, Vol: 10 Issue: 2 ISSN: 0950-4214 Date: June 1996, pp. 117 - 121) In this embodiment, molecular oxygen or peroxide would be introduced in the humidified gas stream at concentrations suitable to result in the formation of enough activated oxygen species on the carbon support that the organic contaminants could be oxidized sufficiently to meet the application requirements.

This specification has been written with reference to various non-limiting and non-exhaustive embodiments. However, it will be recognized by persons having ordinary skill in the art that various substitutions, modifications, or combinations of any of the disclosed embodiments (or portions thereof) may be made within the scope of this specification. Thus, it is contemplated and understood that this specification supports additional embodiments not expressly set forth herein. Such embodiments may be obtained, for example, by combining, modifying, or reorganizing any of the disclosed steps, components, elements, features, aspects, characteristics, limitations, and the like, of the various non-limiting embodiments described in this specification. In this manner, Applicant reserves the light to amend the claims during prosecution to add features as variously described in this specification, and such amendments comply with the applicable description.

Patents, patent applications, publications, scientific articles, books, web sites, and other documents and materials referenced or mentioned herein are indicative of the levels of skill of those skilled in the art to which the inventions pertain, as of the date each publication was written, and all are incorporated by reference as if fully rewritten herein. Inclusion of a document in this specification is not an admission that the document represents prior invention or is prior art for any purpose.

## Claims

1. An active oxidant system comprising:
a macrocyclic tetradentate ligand having the structure 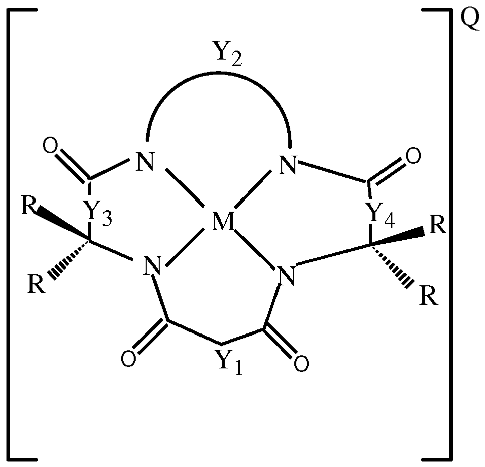 wherein Y₁, Y₃ and Y₄ each represents a bridging group, having zero, one, two or three carbon containing nodes for substitution, and Y₂ is a bridging group having at least one carbon containing node for substitution, each said node containing a C(R) or a C(R)₂ unit and each R substituent is the same or different from the remaining R substituents and ( i ) is selected from the group consisting of alkyl, cycloalkyl, cycloalkenyl, alkenyl, aryl, alkynyl, alkylaryl, halogen, alkoxy, or phenoxy, CH₂CF₃, CF₃ and combinations thereof, or (ii) form a substituted or unsubstituted benzene ring of which two carbon atoms in the ring form nodes in the Y unit, or (iii) together with a paired R substituent bound to the same carbon atom form a cycloalkyl or a cycloalkenyl ring, which may include an atom other than carbon, e.g., cyclopropyl, cyclobutyl, cyclopentyl, or a cyclohexyl ring; M is a transition metal with oxidation states of I, II, III, IV, V, VI, VII or VIII or selected from Groups 3, 4, 5, 6, 7, 8, 9, 10 and 11 of the Periodic Table of the Elements; and, Q is any counterion which would balance the charge of the compound on a stoichiometric basis; **characterized in that** the macrocyclic tetradentate ligand is bound to a carbon-containing support selected from the group consisting of activated carbon, amorphous carbon, and charcoal; and the system further comprises
a source of an oxidant.

2. The active oxidant system recited in claim 1 wherein the oxidant is selected from the group consisting of O-atom transfer oxidants, molecular oxygen, sources of oxygen, ozone, hydrogen peroxide, hydrogen peroxide adducts, t-butyl hydroperoxide, cumyl hydroperoxide, compounds capable of producing hydrogen peroxide in aqueous solution, organic peroxides, perborates, percarbonates, persulfates, perphosphates, persilicates, hypochlorite, peracids, and combinations thereof.

3. The active oxidant system recited in claim 1 or 2 wherein the counterion Q is selected from the group consisting of tetraarylphosphonium, bis- (triphenylphosphorananylidene)-ammonium, and tetraalkylammonium cations.

4. A method for removing oxidizable contaminants from an aqueous medium comprising: exposing an aqueous medium believed to contain oxidizable contaminants to a tetraamido macrocyclic ligand metal catalytic activator bound to a carbon-containing support for a period of time sufficient to oxidize the contaminants, wherein the tetraamido macrocyclic metal ligand comprises a salt of 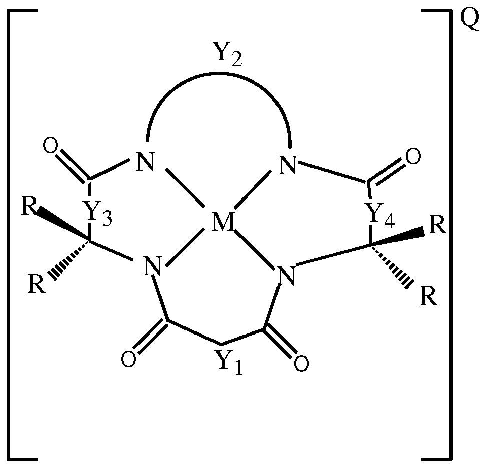 wherein Y₁, Y₃ and Y₄ each represents a bridging group, having zero, one, two or three carbon containing nodes for substitution, and Y₂ is a bridging group having at least one carbon containing node for substitution, each said node containing a C(R) or a C(R)₂ unit and each R substituent is the same or different from the remaining R substituents and ( i ) is selected from the group consisting of alkyl, cycloalkyl, cycloalkenyl, alkenyl, aryl, alkynyl, alkylaryl, halogen, alkoxy, or phenoxy, CH₂CF₃, CF₃ and combinations thereof, or (ii) form a substituted or unsubstituted benzene ring of which two carbon atoms in the ring form nodes in the Y unit, or (iii) together with a paired R substituent bound to the same carbon atom form a cycloalkyl or a cycloalkenyl ring, which may include an atom other than carbon, e.g., cyclopropyl, cyclobutyl, cyclopentyl, or a cyclohexyl ring; M is a transition metal with oxidation states of I, II, III, IV, V, VI, VII or VIII or selected from Groups 3, 4, 5, 6, 7, 8, 9, 10 and 11 of the Periodic Table of the Elements; and, Q is any counterion which would balance the charge of the compound on a stoichiometric basis.

5. The method recited in claim 4 wherein the carbon-containing support is selected from the group consisting of activated carbon, amorphous carbon, and carbon black, charcoal.

6. The method recited in claim 4 wherein the oxidizable contaminants comprise: aromatic groups, conjugated pi systems, natural and synthetic hormones, pesticides, and dyes.

7. The method recited in claim 4 wherein the oxidizable contaminants comprise pathogens.

8. The method of any of claims 4 to 7 further comprising (a) providing a plurality of discrete filtration surfaces spaced along a feed stream, each said filtration surface comprising an tetraamido macrocyclic metal ligand catalytic activator bound to the carbon-containing support; (b) adding an oxidant to the feed stream; and, (c) passing the oxidant containing feed stream through at least one of the filtration surfaces.

9. The method of claim 8, wherein the oxidant is selected from the group consisting of O-atom transfer oxidants, molecular oxygen, sources of oxygen, ozone, hydrogen peroxide, hydrogen peroxide adducts, t-butyl hydroperoxide, cumyl hydroperoxide, compounds capable of producing hydrogen peroxide in aqueous solution, organic peroxides, perborates, percarbonates, persulfates, perphosphates, persilicates, hypochlorite, peracids, and combinations thereof.

10. The method of claim 8 wherein the feed stream flows past the carbon supported catalytic activator.

## Patentansprüche

1. Aktives Oxidanssystem, umfassend:
einen makrozyklischen vierzähnigen Liganden mit der folgenden Struktur 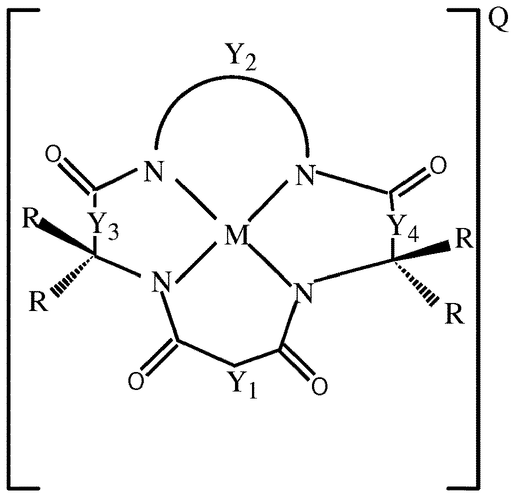 wobei Y₁, Y₃ und Y₄ jeweils eine brückenbildende Gruppe repräsentiert, die null, einen, zwei oder drei Kohlenstoff enthaltende Knoten zur Substitution aufweisen, und Y₂ eine brückenbildende Gruppe mit wenigstens einem Kohlenstoff ist, der Knoten zur Substitution enthält, wobei jeder Knoten eine C(R)- oder eine C(R)₂-Einheit enthält und jeder R-Substituent derselbe oder verschieden von den restlichen R-Substituenten ist und (i) aus der Gruppe selektiert wird, die aus Alkyl, Cycloalkyl, Cycloalkenyl, Alkenyl, Aryl, Alkynyl, Alkylaryl, Halogen, Alkoxy oder Phenoxy, CH₂CF₃, CF₃ und Kombinationen davon besteht oder (ii) einen substituierten oder nicht substituierten Benzenring bilden von dem zwei Kohlenstoffatome im Ring Knoten in der Y-Einheit bilden oder (iii) zusammen mit einem gepaarten R-Substituenten, der an dasselbe Kohlenstoffatom gebunden ist, einen Cycloalkyl- oder einen Cycloalkenylring bilden, der ein Atom anders als Kohlenstoff, z. B., Cyclopropyl, Cyclobutyl, Cyclopentyl oder einen Cyclohexylring einschließen könnte; M ein Übergangsmetall mit Oxidationsstufen von I, II, III, IV, V, VI, VII oder VIII ist oder aus den Gruppen 3, 4, 5, 6, 7, 8, 9, 10 und 11 der Tabelle des Periodensystems der Elemente selektiert wird; und, Q irgendein Gegenion ist, das die Ladung der Zusammensetzung auf einer stöchiometrischen Basis ausgleichen würde; **dadurch gekennzeichnet, dass** der makrozyklische vierzähnige Ligand an einen kohlenstoffhaltigen Träger gebunden ist, der aus der Gruppe selektiert wurde, die aus aktiviertem Kohlenstoff, amorphem Kohlenstoff und Holzkohle besteht; und das System ferner eine Quelle eines Oxidans umfasst.

2. Aktives Oxidanssystem nach Anspruch 1, wobei das Oxidans aus der Gruppe selektiert wird, die aus O-Atom-Transferoxidantien, molekularem Sauerstoff, Sauerstoffquellen, Ozon, Wasserstoffperoxid, Wasserstoffperoxidaddukten, T-Butylhydroperoxid, Cumylhydroperoxid, Zusammensetzungen, die fähig sind, Wasserstoffperoxid in wässriger Lösung, organische Peroxide, Perborate, Percarbonate, Persulfate, Perphosphate, Persilikate, Hypochlorit, Persäuren und Kombinationen davon zu produzieren.

3. Aktives Oxidanssystem nach Anspruch 1 oder 2, wobei das Gegenion Q aus der Gruppe selektiert wird, die aus Tetraarylphosphonium-, Bis- (Triphenylphosphorananyliden)-Ammonium- und Tetraalkylammoniumkationen besteht.

4. Verfahren zum Entfernen von oxidierbaren Kontaminanten aus einem wässrigen Medium, umfassend: ein wässriges Medium von dem angenommen wird, dass es oxidierbare Kontaminanten enthält, wird einem Tetraamido makrozyklischen katalytischen Ligandenmetallaktivator, der an einen kohlenstoffhaltigen Träger gebunden ist, für einen Zeitraum ausgesetzt, der ausreicht die Kontaminanten zu oxidieren, wobei der makrozyklische Tetraamido-Metall-Ligand ein Salz von folgendem umfasst 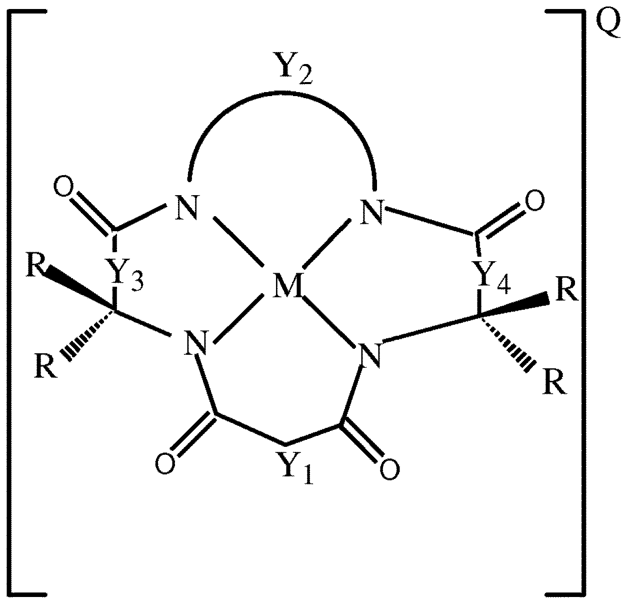 wobei Y₁, Y₃ und Y₄ jeweils eine brückenbildende Gruppe mit null, einem, zwei oder drei kohlenstoffhaltigen Knoten zur Substitution repräsentiert, und Y₂ eine brückenbildende Gruppe ist, die wenigstens einen Kohlenstoff aufweist der einen Knoten zur Substitution enthält, wobei jeder Knoten eine C(R)- oder eine C(R)₂- Einheit enthält und jeder R-Substituent derselbe oder verschieden von den restlichen R-Substituenten ist und (i) aus der Gruppe selektiert wird, die aus Alkyl, Cycloalkyl, Cycloalkenyl, Alkenyl, Aryl, Alkynyl, Alkylaryl, Halogen, Alkoxy oder Phenoxy, CH₂CF₃, CF₃ und Kombinationen davon besteht oder (ii) einen substituierten oder nicht substituierten Benzenring bilden von dem zwei Kohlenstoffatome im Ring Knoten in der Y-Einheit bilden oder (iii) zusammen mit einem gepaarten R-Substituenten, der an dasselbe Kohlenstoffatom gebunden ist, einen Cycloalkyl- oder einen Cycloalkenylring bilden, der ein Atom anders als Kohlenstoff, z. B., Cyclopropyl, Cyclobutyl, Cyclopentyl oder einen Cyclohexylring einschließen könnte; M ein Übergangsmetall mit Oxidationsstufen von I, II, III, IV, V, VI, VII oder VIII ist oder aus den Gruppen 3, 4, 5, 6, 7, 8, 9, 10 und 11 der Tabelle des Periodensystems der Elemente selektiert wird; und, Q irgendein Gegenion ist, das die Ladung der Zusammensetzung auf einer stöchiometrischen Basis ausgleichen würde.

5. Verfahren nach Anspruch 4, wobei der kohlenstoffhaltige Träger aus der Gruppe selektiert wird, die aus aktiviertem Kohlenstoff, amorphem Kohlenstoff und Kohlenschwarz, Holzkohle besteht.

6. Verfahren nach Anspruch 4, wobei die oxidierbaren Kontaminanten umfassen: aromatische Gruppen, konjugierte Pi-Systeme, natürliche und synthetische Hormone, Pestizide und Farbstoffe.

7. Verfahren nach Anspruch 4, wobei die oxidierbaren Kontaminanten Pathogene umfassen.

8. Verfahren nach einem beliebigen der Ansprüche 4 bis 7, das ferner umfasst (a) Bereitstellen einer Vielzahl diskreter Filtrationsflächen, die entlang eines Zuflusses beabstandet sind, wobei jede Filtrationsfläche einen makrozyklischen katalytischen Tetraamido-Metall-Ligandenaktivator umfasst, der an den kohlenstoffhaltigen Träger gebunden ist; (b) Hinzufügen eines Oxidans zum Zufluss; und, (c) Leiten des Oxidans enthaltenden Zuflusses durch wenigstens eine der Filtrationsflächen.

9. Verfahren nach Anspruch 8, wobei das Oxidans aus der Gruppe selektiert wird, die aus O-Atom-Transferoxidantien, molekularem Sauerstoff, Sauerstoffquellen, Ozon, Wasserstoffperoxid, Wasserstoffperoxidaddukten, T-Butylhydroperoxid, Cumylhydroperoxid, Zusammensetzungen, die fähig sind, Wasserstoffperoxid in wässriger Lösung, organische Peroxide, Perborate, Percarbonate, Persulfate, Perphosphate, Persilikate, Hypochlorit, Persäuren und Kombinationen davon zu produzieren.

10. Verfahren nach Anspruch 8, wobei der Zufluss am Kohlenstoff getragenem katalytischen Aktivator vorbeifließt.

## Revendications

1. Système oxydant actif comprenant :
ligand tétradentate macrocyclique de structure 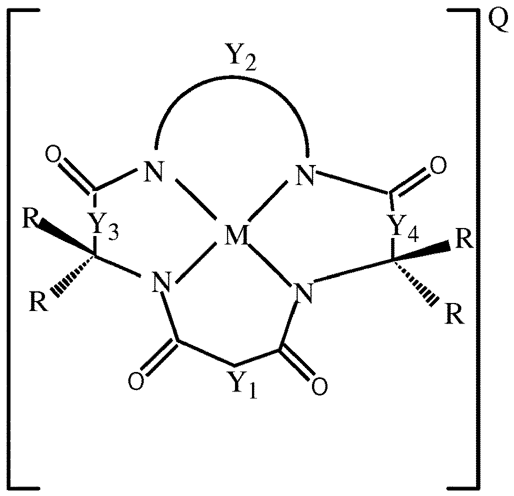
où Y₁, Y₃ et Y₄ représentent chacun un groupe de pontage, comportant zéro, un, deux ou trois noeuds contenant du carbone pour substitution, et Y₂ est un groupe de pontage comportant au moins un noeud contenant du carbone pour substitution, chacun desdits noeuds contenant un motif C(R) ou C(R)₂, et chaque substituant R est identique aux substituants R restant ou différents de ceux-ci et (i) est choisi dans le groupe constitué d'un groupe alkyle, cycloalkyle, cycloalcényle, alcényle, aryle, alcynyle, alkylaryle, halogène, alcoxy ou phénoxy, CH₂CF₃, CF₃ et leurs combinaisons correspondantes, ou (ii) forme un cycle benzène substitué ou non substitué dont deux atomes de carbone du cycle forment des noeuds dans le motif Y, ou (iii) conjointement avec un substituant R apparié lié au même atome de carbone forment un cycle cycloalkyle ou cycloalcényle, qui peut comprendre un atome autre que le carbone, par exemple un cycle cyclopropyle, cyclobutyle, cyclopentyle ou cyclohexyle ; M est un métal de transition dans l'état d'oxydation I, II, III, IV, V, VI, VII ou VIII ou est choisi parmi les groupes 3, 4, 5, 6, 7, 8, 9, 10 et 11 du Tableau périodique des éléments ; et Q est un contre-ion quelconque qui devrait équilibrer la charge du composé sur une base stoechiométrique ; **caractérisé en ce que** le ligand tétradentate macrocyclique est lié à un support contenant du carbone choisi dans le groupe constitué de carbone actif, de carbone amorphe et de charbon de bois ; et le système comprend en outre une source d'un oxydant.

2. Système oxydant actif selon la revendication 1, dans lequel l'oxydant est choisi dans le groupe constitué d'oxydants de transfert d'atomes O, d'oxygène moléculaire, de sources d'oxygène, d'ozone, de peroxyde d'hydrogène, d'adduits de peroxyde d'hydrogène, d'hydroperoxyde de t-butyle, d'hydroperoxyde de cumyle, de composés capables de produire du peroxyde d'hydrogène en solution aqueuse, de peroxydes organiques, de perborates, de percarbonates, de persulfates, de perphosphates, de persilicates, d'hypochlorite, de peracides et de leurs combinaisons.

3. Système oxydant actif selon la revendication 1 ou 2, dans lequel le contre-ion Q est choisi dans le groupe constitué de cations de tétra-arylphosphonium, de bis-(triphénylphosphorananylidène)-ammonium, et de tétra-alkylammonium.

4. Procédé d'élimination de contaminants oxydables d'un milieu aqueux consistant à : exposer un milieu aqueux supposé contenir des contaminants oxydables à un activateur catalytique à ligand métallique tétra-amido macrocyclique lié à un support contenant du carbone pendant une période de temps suffisante pour oxyder les contaminants, le ligand métallique tétra-amido macrocyclique comprenant un sel de 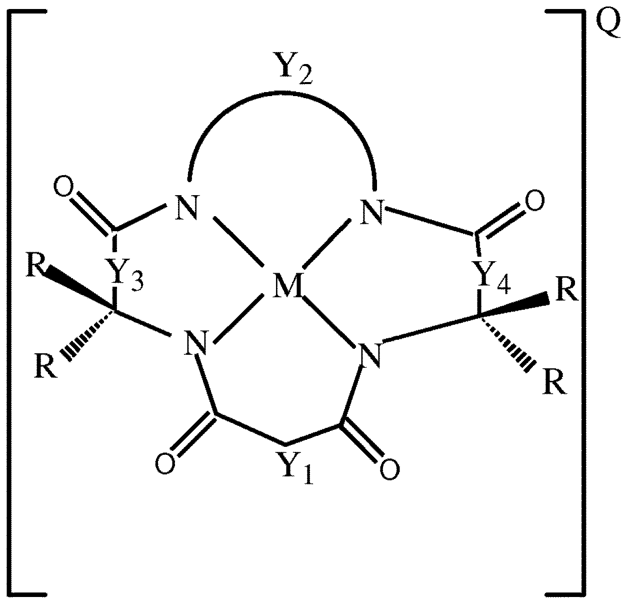 où Y₁, Y₃ et Y₄ représentent chacun un groupe de pontage, comportant zéro, un, deux ou trois noeuds contenant du carbone pour substitution, et Y₂ est un groupe de pontage comportant au moins un noeud contenant du carbone pour substitution, chacun desdits noeuds contenant un motif C(R) ou C(R)₂, et chaque substituant R est identique aux substituants R restant ou différents de ceux-ci et (i) est choisi dans le groupe constitué d'un groupe alkyle, cycloalkyle, cycloalcényle, alcényle, aryle, alcynyle, alkylaryle, halogène, alcoxy ou phénoxy, CH₂CF₃, CF₃ et leurs combinaisons correspondantes, ou (ii) forme un cycle benzène substitué ou non substitué dont deux atomes de carbone du cycle forment des noeuds dans le motif Y, ou (iii) conjointement avec un substituant R apparié lié au même atome de carbone forment un cycle cycloalkyle ou cycloalcényle, qui peut comprendre un atome autre que le carbone, par exemple un cycle cyclopropyle, cyclobutyle, cyclopentyle ou cyclohexyle ; M est un métal de transition dans l'état d'oxydation I, II, III, IV, V, VI, VII ou VIII ou est choisi parmi les groupes 3, 4, 5, 6, 7, 8, 9, 10 et 11 du Tableau périodique des éléments ; et Q est un contre-ion quelconque qui devrait équilibrer la charge du composé sur une base stoechiométrique.

5. Procédé selon la revendication 4, dans lequel le support contenant du carbone est choisi dans le groupe constitué de carbone actif, de carbone amorphe, de noir de carbone et de charbon de bois.

6. Procédé selon la revendication 4, dans lequel les contaminants oxydables comprennent : des groupes aromatiques, des systèmes pi conjugués, des hormones naturelles et synthétiques, des pesticides et des colorants.

7. Procédé selon la revendication 4, dans lequel les contaminants oxydables comprennent des pathogènes.

8. Procédé selon l'une quelconque des revendications 4 à 7, consistant en outre à (a) fournir une pluralité de surfaces de filtration individuelles espacées suivant un flux d'alimentation, chacune desdites surfaces de filtration comprenant un activateur catalytique à ligand métallique tétra-amido macrocyclique lié à un support contenant du carbone ; (b) ajouter un oxydant au flux d'alimentation ; et (c) faire passer le flux d'alimentation contenant l'oxydant à travers au moins une des surfaces de filtration.

9. Procédé selon la revendication 8, dans lequel l'oxydant est choisi dans le groupe constitué d'oxydants de transfert d'atomes O, d'oxygène moléculaire, de sources d'oxygène, d'ozone, de peroxyde d'hydrogène, d'adduits de peroxyde d'hydrogène, d'hydroperoxyde de t-butyle, d'hydroperoxyde de cumyle, de composés capables de produire du peroxyde d'hydrogène en solution aqueuse, de peroxydes organiques, de perborates, de percarbonates, de persulfates, de perphosphates, de persilicates, d'hypochlorite, de peracides et de leurs combinaisons.

10. Procédé selon la revendication 8, dans lequel le flux d'alimentation circule après l'activateur catalytique supporté par le carbone.
